# EUROPEAN PATENT APPLICATION

(11) **EP 4 129 241 A1**
(43) Date of publication of application: **08.02.2023**
(21) Application number: 22188577.5
(22) Date of filing: 03.08.2022
(51) Int. Cl.: A61F 2/30, A61F 2/38

(54) **DYNAMIC BONE LOADING TOTAL KNEE ARTHROPLASTY**

(30) Priority: 03.08.2021 US 202163228757 P
(71) Applicant: Howmedica Osteonics Corp., Mahway, NJ 07430 (US)
(72) Inventor: SERVIDIO, Damon J., TOWACO, 07082 (US)
(74) Representative: Regimbeau

(57) **Abstract**

A joint prosthesis assembly (10, 100) includes a stem (30, 130) that includes a first end (31, 131), a second end (33, 133), and a length that extends between the first and second ends. The stem includes a cylindrical opening (32, 132) that extends into the second end along a portion of the length and terminates within the stem so as to form a base surface (35, 135) that defines an end of the cylindrical opening. The assembly also includes a joint component (20, 120) that has an articular side (26, 126), a bone contact side (28, 128), and a cylindrical boss (22a, 122a) that extends from the bone contact side. The boss is slidingly receivable within the cylindrical opening so that, when the stem and joint component are implanted, the stem is unconstrained in an axial direction and constrained by the stem in a direction transverse to the axial direction.

## Description

### BACKGROUND OF THE INVENTION

Joint arthroplasty is a surgical procedure intended to restore the integrity and functioning of a joint when it becomes damaged, such as by trauma, and/or deteriorated, such as by disease. Joint arthroplasty typically involves resurfacing bones that comprise a joint, such as by cutting, and then applying an artificial implant or prosthesis to the resurfaced bones. Joint prostheses are often secured to underlying bone via bone cement or via a press-fit relationship.

In some circumstances where the underlying bone is compromised, such as in revision procedures or cases of osteoporosis, for example, an intramedullary stem may be used in conjunction with a joint component to provide additional structural support and stability. In this regard, a stem may help distribute loads generated during normal joint functioning over a greater area of bone which can result in a decreased risk of fracture.

However, a problem often encountered with joint component and stem combinations is stress shielding. According to Wolff's law, bone adapts to stress by increasing its density in response to increased levels of stress and decreasing its density in response to a reduction or elimination of stress. Thus, when stress is shielded from a bone, for example, when a stem bears a greater share of load than intended, osteopenia and an associated increased risk of fracture may develop. Therefore, further improvements are desirable.

### BRIEF SUMMARY OF THE INVENTION

Embodiments of joint prosthesis assemblies and methods of their use are described herein. Such assemblies include a joint component and a stem. The joint component is coupled to a stem *in-situ* but in a manner that allows the joint component to move axially relative to the stem while simultaneously being constrained by the stem in a transverse plane. This allows the stem to provide a load bearing function, particularly in the transverse plane. However, the joint component is free to distribute axial load to the end of the bone as it is not inhibited in this regard by the stem. The joint components exemplified in this disclosure are a femoral component and tibial component of a total knee prosthesis. However, while not depicted, other joint components may be utilized in conjunction with an intramedullary stem as described herein. Such joint components may include a femoral head of a hip prosthesis, a glenoid component of a shoulder prosthesis, and a tibial component of an ankle prosthesis, for example.

In one aspect of the present disclosure, a joint prosthesis assembly includes a stem. The stem has a first end, a second end, and a length that extends between the first and second ends. The stem has an opening that extends into the second end along a portion of the length of the stem and terminates within the stem so as to form a base surface that defines an end of the opening. The opening has a constant cross-sectional dimension that extends along a majority of its length from the second end of the stem toward the base surface. The assembly also includes a joint component that has an articular side, a bone contact side, and a boss that extends from the bone contact side of the joint component to a free end of the boss. The boss has a constant cross-sectional dimension along a majority of its length and is slidingly receivable within the opening of the stem such that when the stem and the joint component are implanted, the boss is unconstrained in an axial direction within the opening and constrained by an inner surface of the stem in a direction transverse to the axial direction.

Additionally, the joint component may be a femoral component, and the articular side may include lateral and medial condylar portions. The bone contact side may include a plurality of intersecting planar surfaces. The bone contact side may also include a porous structure configured to promote bone ingrowth. Alternatively, the joint component may be a tibial component, and the articular side may include a tray configured to receive a tibial insert. The bone contact side of the tibial component may include a planar surface and keels extending therefrom.

Continuing with this aspect, the stem may have a length of 75 to 150 mm and may have a porous exterior. The stem may have conical portion and a cylindrical portion. The conical portion may define the first end, and the cylindrical portion may define the second end. Also, an inner surface of the stem may define the opening, and a total gap between the inner surface and the boss may be no more than 2 mm.

Also, the stem and the boss may each include a complementary anti-rotation characteristic at their respective outer and inner surfaces that are configured to engage each other to prevent rotation of the stem and boss relative to each other while permitting axial movement therebetween. The anti-rotation characteristic of the boss may include one or more splines extending radially outwardly from a boss outer surface while also extending axially along a length of the boss. The opening of the stem and the boss may each have a cylindrical cross-sectional geometry. Alternatively, the opening of the stem and the boss may each have a cross-sectional geometry of one of oval, star, square, and triangle.

In another aspect of the present disclosure, a joint prosthesis assembly, includes a stem. The stem has a first end, a second end, and a length that extends between the first and second ends. The stem may have a cylindrical opening that extends into the second end along a portion of the length and terminates within the stem so as to form a base surface that defines an end of the cylindrical opening. The assembly may also include a joint component that has an articular side, a bone contact side, and a cylindrical boss that extends from the bone contact side. The boss is slidingly receivable within the cylindrical opening such that, when the stem and joint component are implanted, the stem is unconstrained in an axial direction and constrained by the stem in a direction transverse to the axial direction.

In a further aspect of the present disclosure, a method of implanting a joint prosthesis, includes inserting a stem into an intramedullary canal of a long bone. The stem has a first end and a second end. The first end has a blind opening that extends therein. The blind opening is cylindrical. After the inserting step, the method includes engaging a joint component to an end of the long bone so that a cylindrical boss extending from the joint component is slidingly received within the blind opening. The cylindrical boss is slidable within the blind opening and constrained by the stem from movement in a direction transverse to a longitudinal axis of the cylindrical boss.

Additionally, the method may include removing a previously implanted prosthesis from an end of the long bone. The joint component may be a femoral component, and the engaging step may include engaging planar resected surfaces of the long bone with corresponding planar surfaces of the femoral component. Also, the engaging step may include inserting pegs of the joint component into the long bone. Further, the engaging step may include interposing bone cement between the planar resected surfaces of the femur and planar surfaces of the femoral component.

### BRIEF DESCRIPTION OF THE DRAWINGS

The features, aspects, and advantages of the present invention will become better understood with regard to the following description, appended claims, and accompanying drawings in which:
FIG. 1 is a side elevational view of a joint prosthesis assembly according to an embodiment of the present disclosure.
FIG. 2 is a side elevational view of joint component of the assembly of FIG. 1.
FIGs. 3A-3F are cross-sectional views of a boss of the joint component of FIG. 2 according to various embodiments of the present disclosure.
FIG. 4A is a side elevational view of an intramedullary stem of the assembly of FIG. 1.
FIG. 4B is a cross-sectional view of the intramedullary stem of FIG. 3A.
FIG. 5 is a cutaway view of the joint prosthesis assembly of FIG. 1 as implanted in a distal femur.
FIG. 6 is a cross-sectional view of a boss of the joint component of FIG. 2 within the intramedullary stem of FIG. 3A.
FIG. 7 is a cutaway view of a joint prosthesis assembly according to another embodiment of the present disclosure.

### DETAILED DESCRIPTION

When referring to specific directions in the following discussion of certain implantable devices, it should be understood that such directions are described with regard to the implantable device's orientation and position during exemplary application to the human body. Thus, as used herein, the term "proximal" means close to the heart and the term "distal" means more distant from the heart. Also, as used herein, the terms "about," "generally" and "substantially" are intended to mean that slight deviations from absolute are included within the scope of the term so modified.

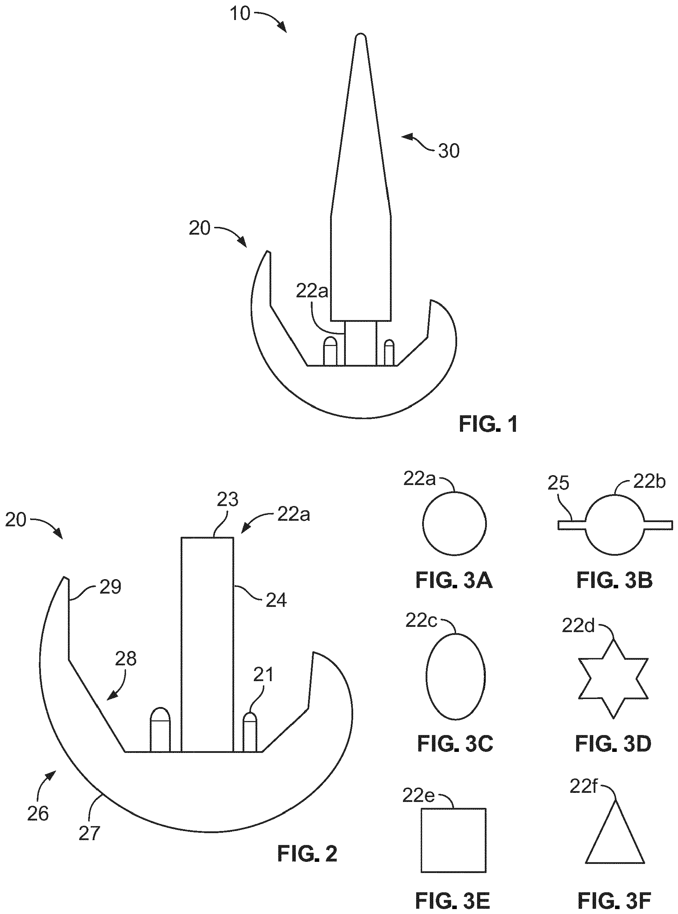

FIGs. 1-6 depict a joint prosthesis assembly 10 according to an embodiment of the present disclosure. Assembly 10 generally includes a joint component 20 and an intramedullary stem 30.

Joint component 20, as shown, is a femoral component of a total knee prosthesis. Femoral component 20 includes an articular side 26, a bone contact side 28, and a boss 22a. Articular side 28 includes condyles 27 for articulating with a tibial component. The bone contact side 28 includes a plurality of planar intersecting surfaces 29 which can include three, four, or five intersecting planar surfaces 29, for example. However, in other embodiments, bone contact side 28 may include one or more curved surfaces or a combination of planar and curved surfaces. In addition, bone contact side 28 may include a porous structure configured to promote bone ingrowth. Alternatively, femoral component 20 may include corrugations or the like to aid in cement adherence. Thus, femoral component 20 can be configured to be press-fit or cemented to a distal femur.

Boss 22a extends from bone contact side 28 to a free end 23 and is cylindrical. Thus, its cross-sectional shape is circular with a constant diameter along its length, as best shown in FIGs. 2 and 3A. In other words, boss 22a has a constant cross-sectional dimension along its entire length. However, boss 22a may not be cylindrical along its entire length but rather may be cylindrical along the majority of its length while being conical along a minority of its length. In other words, boss 22a may have a constant cross-sectional dimension along a majority of its length, and a tapering cross-sectional dimension along a minority of its length. It should be noted that that in such embodiment, the cylindrical portion (i.e., the constant dimension portion) extends from a free end 23 of boss 22a to a junction with the conical portion (i.e., the tapering cross-sectional portion), while the conical portion may extend from the bone contact side of femoral component 20 to the junction. Such configuration allows boss 22a to move unconstrained in an axial direction within an opening 32 of stem 30 to promote subsidence and maximal force application at an end of the bone to prevent stress shielding.

Boss 22a may also include one or more anti-rotation features extending along at least a portion of its length. For example, boss 22a may include one or more splines 25, as shown in FIG.3B, that extend radially outwardly from a boss outer surface 24 while also extending axially along a length of boss 22a.

Boss 22a, however, may be constructed to have other cross-sectional shapes. For example, various shapes that can comprise the cross-sectional geometry of boss 22a include an oval 22c (FIG. 3C), a star 22d (FIG. 3D), and a polygon, such as a square 22e (FIG. 3E), triangle 22f (FIG. 3F), and the like. Such stem shapes have inherent anti-rotation characteristics in that they each include at least one cross-sectional dimension greater than another within the same cross-sectional plane. Also, as explained further below, it is desirable that any one of these bosses 22a-f be capable of sliding within stem 30. Thus, it is preferable that the cross-sectional dimensions of each boss 22a-f be constant along their respective lengths regardless of the cross-sectional shape selected.

In addition to boss 22a, other features may extend from bone contact side 28. For example, one or more pegs 21 may extend from bone contact side 28, as shown in FIG. 2. Such pegs 21 may be inserted into corresponding openings in a bone to help prevent rotation of femoral component 20 relative to the bone.

Stem 30 includes first and second ends 31, 33 and a length that extends therebetween. The length of stem 30 is preferably 75 to 100 mm. In this regard, stem 30 is configured to be implanted into a metaphysis of a long bone and to extend from the metaphysis into the diaphysis of the bone. An exterior of stem defines a cylindrical portion 34 and a conical portion 36. However, in some embodiments, stem 30 may be conical along its entire length. The conical shape of conical portion 36 forms a taper configured to be press-fit into an intramedullary canal of a bone. In some embodiments, stem 30 may have a porous structure on its exterior for bone ingrowth and/or may include a plurality of splines for anti-rotation.

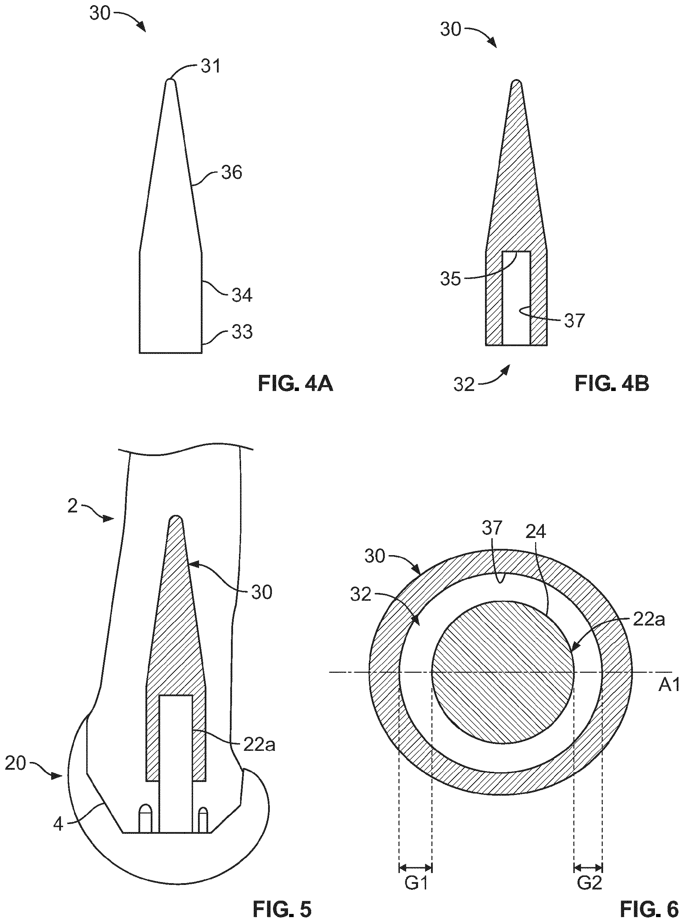

An inner surface 37 of stem 30 defines a blind opening 32 that extends axially through second end 33 of stem 30 and terminates within stem 30 at a base surface 35, as shown in FIG. 4B. Opening 32 has a shape complementary to that of any one of bosses 22a-f of femoral component 20. Thus, in embodiments in which femoral component includes cylindrical boss 22a, such as that shown in FIGs. 2 and 3A, opening 32 is correspondingly cylindrical. In other words, opening 32 may have a constant cross-sectional dimension along its entire length or along a majority of its length with a tapering cross-sectional dimension along a minority of its length. Similarly, where boss has any of the shapes shown in FIGs. 3B-3F, opening 32 has the same shape. However, opening 32 is slightly larger in cross-sectional dimension than that of the corresponding boss 22a-f so that it can slide freely into opening 32. In this regard, the cross-sectional dimension of opening 32 is such that a total transverse gap is formed between inner surface 37 of stem 30 and outer surface 24 of boss 22a-f that is no more than 2 mm. For example, as shown in FIG. 6, opening of stem 32 is sized relative to boss 22a such that gaps G1 and G2 between outer surface 24 of boss 22a and inner surface 37 of stem 30 along a transverse axis A1 creates a total gap Gₜₒₜₐₗ of a maximum 2 mm which is the sum of gaps G1 and G2. Thus, when boss 22a is received within opening 32, boss 22a is unconstrained in an axial direction while being constrained in a direction transverse to a longitudinal axis of boss 22a.

A method of use is now described in the context of a revision procedure. In a revision procedure, a previously implanted femoral component is removed from a distal end of bone 2. Bone 2 may then be prepared by cutting its distal end to correspond to the bone contact side 28 of femoral component 20. In addition, the intramedullary canal of femur 2 may be prepared by reaming, broaching, or the like to accommodate stem 30.

Once bone 2 is prepared, stem 30 is inserted into the intramedullary canal in a press-fit manner which is facilitated by the conical taper of stem 30. Thereafter, femoral component 20 is placed onto the distal end femur 2 so that bone contact side 28 seats onto femur 2 either in a press-fit manner or via a layer of bone cement. As femoral component 20 is placed onto femur 2, boss 22a is slid into opening 32 of stem as shown in FIGs. 5 and 6. Due to the corresponding geometries of boss 22a and stem opening 32, boss 22a axially slides into opening so that it is not constrained from axial movement. This allows femoral component 20 to fully seat on femur 2 and for axial loads applied to femoral component 2 to be transferred to the distal femur through bone contact side 28 of component 20. However, stem 30 constrains boss 22a in a transverse direction relative to a longitudinal axis of boss 22a. Thus, loads that act transverse to boss 22a during normal functioning of a knee joint are shared by femoral component 20 and stem 30.

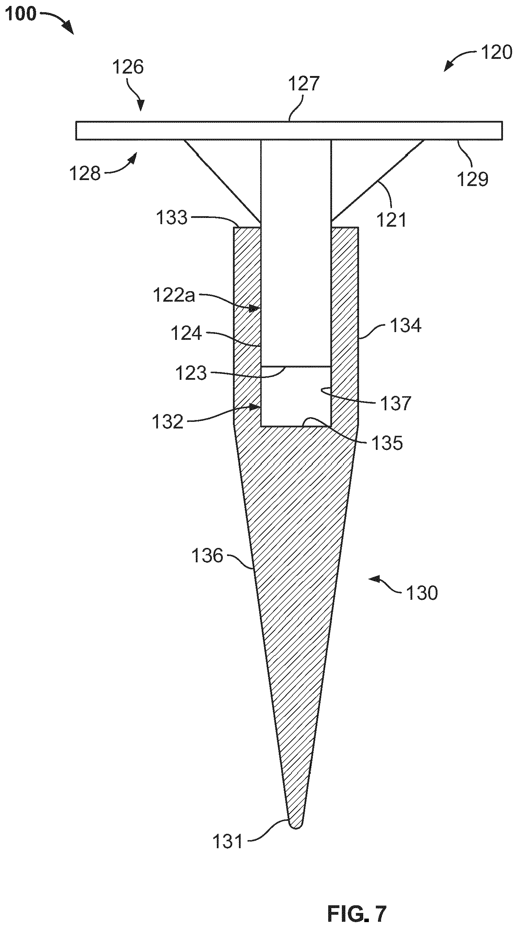

FIG. 7 depicts another joint prosthesis assembly 100. For ease of review, like elements will be accorded like reference numerals to that of joint prosthesis assembly 10 but within the 100-series of numbers. For instance, joint prosthesis assembly 100 includes a joint component 120 with a boss 122a and a stem 130 with a complementary opening defined by an inner surface 131 as previously described. In this regard, boss 122a of joint component 120 is axially slidable within stem 130 while being constrained in a transverse direction. However, assembly 100 also differs from assembly 10 in that joint component 120 is a tibial component and, more specifically, a tibial baseplate. Tibial baseplate 120 generally includes an articular side 126 and a bone contact side 128. Articular side 126 of baseplate 120 includes a tray 127 configured to receive a polymer insert, as known in the art, for articulation with a femoral component, such as femoral component 20. In addition, bone contact side 128 of baseplate includes a planar surface 129 that may include a porous structure for bone ingrowth. Alternatively, tibial component 120 may include corrugations or the like to aid in cement adherence. Thus, tibial component 120 can be configured to be press-fit or cemented to a distal femur. Additionally, tibial component 120 may include one or more keels 121 extending therefrom for anti-rotation within a bone, as shown.

In use, just as with assembly 10, stem 130 is first implanted into a proximal tibia and then tibial component 120 is implanted so that baseplate 120 fully seats on a proximal tibial plateau while boss 122a slidably extends within the opening of stem 130. In this regard, axial loads applied to baseplate 120 can be fully transferred to the underlying bone while transverse loads are alleviated by stem 130.

Although the embodiments described herein are exemplified in the form of a femoral component and tibial component of a total knee prosthesis, it should be understood that principles described herein can be applied to joint prostheses of various kinds, such as a hip, shoulder, and ankle prostheses.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A joint prosthesis assembly (10, 100), comprising:
a stem (30, 130) having a first end (31, 131), a second end (33, 133), and a length extending between the first and second ends (31, 131,33, 133), the stem (30) having an opening (32, 132) extending into the second end (33, 133) along a portion of the length of the stem (30, 130) and terminating within the stem (30, 130) so as to form a base surface (35, 135) defining an end of the opening (32, 132), the opening (32, 132) having a constant cross-sectional dimension extending along a majority of its length from the second end (33, 133) of the stem (30, 130) toward the base surface (35, 135); and
a joint component (20, 120) having an articular side (26, 126), a bone contact side (28, 128), and a boss (22a, 122a) extending from the bone contact side (28, 128) of the joint component (20, 120) to a free end (23, 123) of the boss (22a, 122a), the boss (22a, 122a) having a constant cross-sectional dimension along a majority of its length and being slidingly receivable within the opening (32, 132) of the stem (30, 130),
wherein, when the stem (30, 130) and the joint component (20, 120) are implanted, the boss (22a, 122a) is unconstrained in an axial direction within the opening (32, 132) and constrained by an inner surface (37, 137) of the stem (32, 132) in a direction transverse to the axial direction.

2. The joint prosthesis assembly (10, 100) of claim 1, wherein the joint component (20, 120) is a femoral component (20), and the articular side (28) includes lateral and medial condylar portions (27).

3. The joint prosthesis assembly (10, 100) as in claim 2, wherein the bone contact side (28) includes a plurality of intersecting planar surfaces (29).

4. The joint prosthesis assembly (10, 100) as in any preceding claim, wherein the bone contact side (28, 128) includes a porous structure configured to promote bone ingrowth.

5. The joint prosthesis assembly (10, 100) as in any preceding claim, wherein the stem (30, 130) has a length of 75 to 150 mm.

6. The joint prosthesis assembly (10, 100) as in any preceding claim, wherein the stem (30, 130) has a porous exterior.

7. The joint prosthesis assembly (10, 100) as in any preceding claim, wherein the stem (30, 130) has conical portion (36, 136) and a cylindrical portion (34, 134), the conical portion (36, 136) defining the first end (31, 131), the cylindrical portion (34, 134) defining the second end (33, 133).

8. The joint prosthesis assembly (10, 100) as in any preceding claim, wherein the joint component (20, 120) is a tibial component (120), and the articular side (126) includes a tray (127) configured to receive a tibial insert.

9. The joint prosthesis assembly (10, 100) as in claim 8, wherein the bone contact side (128) of the tibial component (120) includes a planar surface (129) and keels (121) extending therefrom.

10. The joint prosthesis assembly (10, 100) as in any preceding claim, wherein an inner surface (37, 137) of the stem (30, 130) defines the opening (32, 132), and a total gap (Gₜₒₜₐₗ) between the inner surface (37, 137) and the boss (22a, 122a) is no more than 2 mm.

11. The joint prosthesis assembly (10, 100) as in any preceding claim, wherein the stem (30, 130) and the boss (22a, 122a) each include a complementary anti-rotation characteristic (25, 22c-f) at their respective outer and inner surfaces (24, 124, 37, 137) that are configured to engage each other to prevent rotation of the stem (30, 130) and boss (22a, 122a) relative to each other while permitting axial movement therebetween.

12. The joint prosthesis assembly (10, 100) of claim 11, wherein the anti-rotation characteristic (25, 22c-f) of the boss (22a, 122a) includes one or more splines (25) extending radially outwardly from a boss outer surface (24, 124) while also extending axially along a length of the boss (22a, 122a).

13. The joint prosthesis assembly (10, 100) as of any of the preceding claims, wherein the opening (32, 132) of the stem (30, 130) and the boss (22a, 122a) each have a cylindrical cross-sectional geometry.

14. The joint prosthesis assembly (10, 100) as of any of claims 1-12, wherein the opening (32, 132) of the stem (30, 130) and the boss (22a, 122a) each have a cross-sectional geometry of one of oval (22c), star (22d), square (22e), and triangle (22f).
